# EUROPEAN PATENT SPECIFICATION

(11) **EP 4 342 397 B1**
(45) Date of publication and mention of the grant of the patent: **11.03.2026**
(21) Application number: 21740110.8
(22) Date of filing: 17.05.2021
(51) Int. Cl.: A61B 17/34, A61B 34/20, A61B 18/20, A61B 90/00, A61B 34/30

(54) **LASER CUTTING SURGICAL SYSTEM WITH SURGICAL ACCESS PORT TRACKING**
CHIRURGISCHES LASERSCHNEIDSYSTEM MIT VERFOLGUNG EINES CHIRURGISCHEN ZUGANGSPORTS
SYSTÈME CHIRURGICAL DE COUPE AU LASER AVEC SUIVI D'ORIFICE D'ACCÈS CHIRURGICAL

(43) Date of publication of application: 27.03.2024
(73) Proprietor: Deneb Medical, S.L., 20014 Donostia - San Sebastián (ES)
(72) Inventor: RUBIO ZAMORA, Oliver, 20014 Donostia-San Sebastián (ES); OTEGUI ARRUTI, Asier, 20014 Donostia-San Sebastián (ES); ORTEGA QUIJANO, Noé, 20014 Donostia-San Sebastián (ES); ARREGUI ALTUNA, Juan, 20014 Donostia-San Sebastián (ES)
(74) Representative: ABG Intellectual Property Law, S.L.
(86) International application number: PCT/ES2021/070352
(87) International publication number: WO 2022/243577

(56) References cited:
- US-A1- 2017 265 947
- US-A1- 2018 055 578
- US-A1- 2020 198 147

## Description

### OBJECT OF THE INVENTION

The present invention provides a laser cutting surgical system capable of aligning the laser beam based on the three-dimensional pose of the surgical access port. In particular, the invention is suited for robot-assisted surgical interventions.

### BACKGROUND OF THE INVENTION

In robot-assisted minimally invasive surgeries (MIS), robots often use laser-based cutting systems. The laser beam gradually converges from its output channel to a focal position in which it acts in an optimal manner, i.e., to the position in which ablation effectiveness is maximum.

In surgeries of this type, the laser strikes the patient through so-called access ports or trocars. Surgical instruments of this type carry out a purely mechanical function: they define the surgical field and the direction in which the robot is to act, keeping the patient's skin and other soft tissues out of the surgical field so as not to interfere with the work of the robot.

For a robotic system to work suitably, the robot must be aligned with the surgical access port. Otherwise, the laser beam intersects the trocar, causing the effective power of the laser to be reduced at the focal position, and consequently, reducing the effectiveness of the cut. Furthermore, the current trend in MIS is to use trocars with increasingly smaller diameters so as to minimize, as much as possible, the invasiveness of the operation; therefore, the alignment between the laser of the robotic system and the body of the trocar which ensures that the entire beam strikes the target tissues represents an increasingly greater challenge.

In US 2018/055578 A1, a medical navigation system is provided, comprising a computing device having a processor coupled to a memory, a tracking camera for tracking medical devices, and a display for displaying an image; an automated arm assembly electrically coupled to the computing device and controlled by a signal provided by the computing device, the automated arm assembly including a multi-joint arm having a distal end connectable to an effector that supports a surgical camera electrically coupled to the computing device; and a medical device having a tracking marker attachable to the medical device. The computing device is configured to position the automated arm assembly, based on an input command, in response to a position in space of the medical device such that a surgical site of interest remains within a field of view of the surgical camera, the position in space of the medical device determined by the computing device based on a signal provided to the computing device by the tracking camera; and display on the display an image provided by an image signal generated by the surgical camera.

### DESCRIPTION OF THE INVENTION

The present invention proposes a solution to the aforementioned problems by means of a laser cutting surgical system according to claim 1. The dependent claims define preferred embodiments of the invention. In

a first aspect, there is provided *a surgical access port suitable for surgical interventions, comprising:*
- *a main body configured as a tubular conduit, with an empty inner space therein and open at the ends of the tubular conduit, and*
- *a first plurality of fiducial markers, spaced from one another, and fixed to the main body in a rigid manner.*

Throughout the description, a surgical access port or trocar will be understood to mean an instrument used for defining the surgical field in minimally invasive surgeries. Surgical instruments of this type also define the direction in which the operation is performed, keeping the patient's skin and other soft tissues out of the surgical field.

The trocar according to the first aspect comprises a tubular main body with two openings at its ends. The surgical instrument is introduced through the first opening, going through the empty inner space of the trocar, and finally coming into contact with the patient's tissues by going through the second opening. In the context of the invention, the surgical instrument is a laser beam for cutting tissues.

The alignment of the trocar with the laser beam requires knowing the exact three-dimensional pose of said trocar. Throughout the document, three-dimensional pose of an element will be understood to mean a set of parameters which allow said element to be positioned in space with six degrees of freedom. In particular, the three-dimensional pose comprises the three-dimensional position and orientation of the element.

In order to achieve said alignment, the trocar of the first aspect comprises a plurality of fiducial markers fixed to the tubular body in a rigid manner. Said markers must be separated from one another such that they can be visualized in space individually. Throughout the document, a fiducial marker will be understood to mean an element to be fixed in an object and in a specific position with respect to said object, in this case the trocar, which serves as a reference point and which, with three or more, allow the three-dimensional pose of said object to be determined. Preferably, each of the fiducial markers is a sphere.

In a second aspect, there is provided *a positioning system for a surgical environment, comprising:*
- *a surgical access port according to the first inventive aspect;*
- *a tracking device adapted for establishing the three-dimensional pose in space of the first plurality of fiducial markers of the surgical access port;*
- *a central processing unit adapted for:*
   *storing a reference coordinate system;*
   *receiving the three-dimensional pose of the first plurality of fiducial markers from the tracking device;*
   *determining at least the three-dimensional pose of the empty inner space of the main body of the surgical access port with respect to the reference coordinate system based on the three-dimensional pose of the first plurality of fiducial markers; and*
   *storing said three-dimensional pose of the empty inner space of the main body of the surgical access port with respect to the reference coordinate system.*

In this second aspect, there is provided a positioning system which comprises the trocar with fiducial markers of the first inventive aspect and is capable of positioning and orienting said trocar in a three-dimensional manner. To that end, it further comprises a tracking device located such that it visualizes the fiducial markers of the trocar, and a central processing unit in communication with the tracking device with the capacity to receive, emit, store, and process data. Preferably, the central processing unit comprises a processor or a microprocessor.

Throughout the document, tracking device will be understood to mean a device used for observing one or more stationary or moving objects and for providing the three-dimensional pose thereof over time. The tracking device according to the second aspect observes the fiducial markers of the trocar and provides to the central processing unit the three-dimensional pose thereof. Preferably, the tracking device is an optical tracking device.

In turn, the central processing unit receives this information and processes it for positioning and orienting the trocar in space in a three-dimensional manner with respect to a previously stored reference coordinate system. In particular, the central processing unit determines the three-dimensional pose of the empty inner space of the main body of the surgical access port with respect to the reference coordinate system based on the three-dimensional pose of the fiducial markers.

In a particular example, the central processing unit determines the three-dimensional pose of the empty inner space of the main body based on an actuation path. This actuation path, which is a segment of a straight line, must go through the empty inner space of the main body of the access port, from end to end, without intersecting said main body at any point. This path will be understood to mean one of the possible paths a cutting laser beam will follow along the surgical access port in the operating mode; the laser beam will thus go through the main body of the trocar without intersecting it. According to a preferred example, the actuation path is chosen as the straight line segment farthest way from the internal walls of the trocar in order to have the largest safety margin.

In order to be able to calculate the three-dimensional pose of the empty inner space of the main body in space, the central processing unit previously knows the location of the fiducial markers in the geometry of the trocar, preferably because a prior calibration has been performed or because the trocar has been designed and manufactured according to a predetermined configuration. Next, the tracking device determines the three-dimensional pose of the fiducial markers with respect to the reference coordinate system, said reference coordinate system being stored by the central processing unit, and sends this information to the central processing unit. Lastly, by matching up points, the central processing unit calculates the translation and rotation needed to convert the coordinates of the fiducial markers with respect to the reference coordinate system, as it is in this reference coordinate system where the coordinates of the empty inner space of the main body are expressed. The central processing unit thereby establishes the three-dimensional pose of the empty inner space of the trocar from the position of the fiducial markers expressed in the reference coordinate system.

Lastly, the central processing unit stores the three-dimensional pose of the empty inner space of the main body.

In a third aspect, there is provided *a laser cutting surgical system, comprising:*
- *a positioning system for a surgical environment according to the second inventive aspect;*
- *a laser cutting surgical robot comprising a laser cutting module adapted for emitting a laser beam acting throughout the empty inner space of the main body of the surgical access port,*
*wherein the central processing unit is further adapted for:*
*determining, based on the three-dimensional pose of the empty inner space of the main body, at least one actuation direction of the laser beam such that the laser beam in the at least one actuation direction goes through the inner space of the main body without intersecting said main body;*
*positioning and orienting the laser cutting module for the laser beam to be oriented according to the at least one determined actuation direction.*

In this third aspect, there is provided the complete laser cutting system comprising the positioning system described above and a surgical robot capable of cutting tissues by laser. As previously mentioned, the laser beam must go through the empty space of the main body of the surgical access port which has been positioned and oriented in a three-dimensional manner by means of the positioning system of the second aspect.

Throughout the document, laser beam will be understood to mean a plurality of laser rays converging according to a central direction and the energy of which is confined in a volume oriented according to said direction. Thus, in the context of the invention, no point of the volume formed by the laser beam intersects the main body of the trocar in the operating mode.

In particular, the laser beam is emitted from a laser cutting module comprised in the robot. Throughout the description, the laser cutting module will be understood to comprise a laser emitter which is preferably an Er:YAG laser emitter with an emission wavelength of 2940 nanometers.

The laser beam of the robot must be perfectly aligned with the trocar in order to perform cutting. This alignment is achieved as a result of the central processing unit, which determines at least one actuation direction of the laser beam for said laser beam to go through the empty inner space of the main body in the operating mode without intersecting the walls of said main body. The central processing unit determines the actuation direction or directions taking into account, in addition to the three-dimensional pose of the empty inner space of the main body, the shape of said empty inner space of the main body and the volume formed by the laser beam. Thus, when the laser beam acts according to the different determined actuation directions, said laser beam strikes an entire region of tissue to be cut without intersecting the main body of the trocar at any point.

Lastly, once the actuation direction or directions have been determined, the central processing unit positions and orients the laser in a three-dimensional manner according to said actuation direction or directions. If there is more than one actuation direction, positioning and orientation are performed according to a determined sequence of actuation directions.

In an example in which a particular actuation path through the empty inner space of the main body has been determined, the central processing unit determines the actuation direction of the laser beam such that said actuation direction contains the actuation path. Additionally, other possible actuation directions will contain segments parallel to said actuation path provided that the requirement of the laser beam not intersecting the walls of the main body at any point is fulfilled.

In one embodiment, the tracking device is positioned on the cutting module of the surgical robot and is capable of determining the three-dimensional pose of said module. The tracking device thus provides this information to the central processing unit and said unit determines the relative three-dimensional pose of the laser cutting module with respect to the main body of the trocar.

Based on this relative three-dimensional pose of the cutting module with respect to the main body of the trocar, the central processing unit calculates an error in the alignment of both elements. If said error exists, the central processing unit repositions and/or reorients the laser cutting module for the laser beam to be oriented according to the at least one actuation direction it has previously determined.

In a particular example, if the alignment error exceeds a limit defined as being acceptable, the central processing unit gives off an alarm. Preferably, an alignment is considered to be unacceptable when the laser beam intersects the main body of the trocar at any point. In another particular example, which is an alternative to or simultaneous with the preceding example, the central processing unit gives off an alarm when it detects that the alignment error cannot be corrected, for example, because there is a failure in communication between said unit and the robot. Preferably, under these circumstances the emission of the laser is stopped.

In one embodiment:
- *the laser cutting surgical robot further comprises a second plurality of fiducial markers,*
- *the tracking device is further adapted for establishing the three-dimensional pose in space of the second plurality of fiducial markers; and*
- *the central processing unit is further adapted for:*
   *receiving the three-dimensional pose of the second plurality of fiducial markers from the tracking device;*
   *determining at least the relative three-dimensional pose between the laser cutting module and the empty inner space of the main body of the surgical access port based on the three-dimensional pose of the second plurality of fiducial markers; and*
   *storing said relative three-dimensional pose between the laser cutting module and the empty inner space of the main body of the surgical access port.*

In this embodiment, the surgical robot comprises its own fiducial markers which are visible for the tracking device. Preferably, the tracking device is not mounted on the cutting module but rather is located in space such that it can simultaneously visualize the fiducial markers of the trocar and of the robot at the same time.

The tracking device establishes the three-dimensional pose of the fiducial markers of the robot and sends this information to the central processing unit, which subsequently determines the relative three-dimensional pose of the laser cutting module and the main body of the trocar, in particular, of the empty inner space of said main body, based on said information. Lastly, the central processing unit stores this relative three-dimensional pose.

Based on this relative three-dimensional pose of the cutting module with respect to the main body of the trocar, the central processing unit calculates an error in the alignment of both elements. If said error exists, the central processing unit repositions and/or reorients the laser cutting module for the laser beam to be oriented according to the at least one actuation direction previously determined by the central processing unit itself.

In a particular example, if the alignment error exceeds a limit defined as being acceptable, the central processing unit gives off an alarm. Preferably, an alignment is considered to be unacceptable when the laser beam intersects the main body of the trocar at any point. In another particular example, which is an alternative to or simultaneous with the preceding example, the central processing unit gives off an alarm when it detects that the alignment error cannot be corrected, for example, because there is a failure in communication between said unit and the robot. Preferably, under these circumstances the emission of the laser is stopped.

In one embodiment, *the central processing unit generates a numerical model comprising at least:*
- *the shape of the empty inner space of the main body of the surgical access port;*
- *the coordinates of the first plurality of fiducial markers with respect to the reference coordinate system and the relationship of coordinates and orientation between the empty inner space of the main body of the surgical access port and the first plurality of fiducial markers;*
- *if the system comprises the second plurality of fiducial markers, the coordinates of the second plurality of fiducial markers with respect to the reference coordinate system and the relationship of coordinates and orientation between the at least one actuation direction of the laser beam and the second plurality of fiducial markers.*

All the mathematical estimates calculated by the central processing unit are based on the data of the numerical model generated by said unit. This numerical model allows determining whether or not the laser beam and the main body of the trocar are aligned as well as whether said laser beam intersects the main body of the trocar at any point based on the shape of the empty inner space of the main body of the trocar and on the relative positions of the laser cutting module and the main body of the trocar. These relative positions of the laser cutting module and the main body of the trocar are established through an intermediate kinematic chain, such that the configuration of the trocar, and in particular, the three-dimensional pose of the empty inner space, refer to the fiducial markers of the trocar through the numerical model, and the three-dimensional pose of the laser beam of the laser cutting module refer to the fiducial markers of the cutting module also through the numerical model.

Therefore, the central processing unit only requires relating the position of the first plurality of fiducial markers and the position of the second plurality of fiducial markers in order to be able to relate the relative position of any point of the laser cutting module with any point of the trocar. In particular, if these elements are misaligned, the central processing unit estimates the three-dimensional repositioning and/or reorientation that need to be applied to the laser cutting module for the laser to be oriented according to the at least one previously determined actuation direction.

Moreover, as mentioned, the numerical model comprises information about the shape, that is, width, height, and depth, of the empty inner space of the main body. These dimensions define the surgical region, i.e., they allow the central processing unit to know how much space is available for the laser to be able to act inside the trocar, and consequently, for being able to calculate the actuation direction or directions.

In one embodiment, *the central processing unit determines the three-dimensional pose of the empty inner space of the main body of the surgical access port by means of the geometric relationships of the numerical model.* In another embodiment, *the geometric relationships between points of the numerical model are determined by means of a hand-eye calibration, by means of a calibration based on geometric restrictions, by means of direct measurement performed by a metrology system or a calibration device, or by means of an in vivo calibration using a tracked probe.*

As previously mentioned, as a result of the numerical model, the central processing unit estimates the relative positions between the main body of the trocar (in particular its empty inner space) and the laser cutting module which are comprised in the laser cutting surgical system. These estimations are based on the three-dimensional pose of the fiducial markers of the trocar and of the robot, if there are any.

The geometric relationships which allow the three-dimensional pose of the laser cutting module of the robot to be estimated can be determined by means of different calibration techniques, such as hand-eye calibration, calibration based on geometric restrictions, or direct measurement by means of external elements with respect to the system, such as a metrology system or a calibration device.

Hand-eye calibration, which can be performed when the laser cutting surgical robot comprises a robotic arm, consists of calculating the 3D transformation existing between the coordinate system of the robotic arm ("hand") and a tracking camera ("eye"), or tracking device in the context of the invention, by solving the AX=ZB system of equations, wherein A and B are the coordinate systems of the robotic arm and of the tracking camera, respectively, and wherein X is the transformation of the three-dimensional pose relating an arbitrary position with the coordinate system of the robotic arm and Z is the transformation of the three-dimensional pose relating the same arbitrary position with the coordinate system of the tracking camera. Said arbitrary position is determined by the three-dimensional pose in space of the plurality of fiducial markers of the robot. In order to solve said system of equations, a series of movements of the robotic arm is performed, for which movements the resulting positions and orientations both of the kinematics of the robotic arm and of the fiducial markers detected by the camera are stored, in order to thus construct the equations of the system based on a geometric interpretation, and then solve said system by means of an optimization method.

Calibration based on geometric restrictions consists of estimating the mathematical equations which determine the relationship between the laser cutting surgical robot and its fiducial markers based on specific movements measured at the same time by the kinematics of the robot and by the tracking device.

Moreover, the geometric relationships which allow the three-dimensional pose of the main body of the trocar to be estimated can be determined by means of an *in vivo* calibration by mounting and removing the fiducial markers on/from the trocar as needed, using a tracked probe for that purpose.

Tracked probe will be understood to mean a device that is rigid enough so as not to be deformed when pressing on the main body of the trocar and has a termination at one end for sensing specific physical positions of the main body in a consistent manner. Preferably, the termination is a conical tip. The tracked probe further comprises a series of fiducial markers which can be located in space by means of the tracking device which further knows the relative three-dimensional poses between said markers and the termination of the probe used for sensing. In particular, these relative three-dimensional poses are known as a result of a prior calibration process or as a result of the probe, including its termination and markers, having been manufactured according to a known predefined design. By matching up points, the tracking device thereby estimates the three-dimensional pose of the termination of the probe in the reference coordinate system using the fiducial markers of said probe, and therefore estimates the three-dimensional pose of the sensed points, which will serve as fiducial markers of the trocar, in the reference coordinate system. Preferably, the trocar comprises a plurality of holes in known positions in which the tracked probe is introduced in order to sense them and to be able to serve as fiducial markers of the trocar.

In one embodiment, *the tubular conduit of the main body of the surgical access port is cylindrical.*

Preferably, the actuation direction of the laser beam or one of the actuation directions determined by the central processing unit contains the axis of revolution of the cylinder. Advantageously, the position thereof coinciding with the axis of revolution maintains the largest possible distance to the walls of the trocar, thus increasing the level of safety in case of a misalignment between the trocar and the laser beam that cannot be corrected with a sufficient response time.

Optionally, if the central processing unit has determined more than one actuation direction, the remaining actuation directions contain segments parallel to the axis of revolution of the cylinder. Alternatively, the remaining actuation directions contain segments not parallel to the axis of revolution of the cylinder which go through said cylinder from end to end. In either of the two cases, the laser beam following one of the actuation directions goes longitudinally through the inside of the trocar without intersecting the main body at any point.

In one example, the central processing unit determines an actuation path which represents the passage of the laser beam through the empty space of the main body extending along the axis of revolution of the cylinder. In an alternative example, the actuation path extends along a segment parallel to the axis of revolution of the cylinder, said segment going through the cylinder from end to end without intersecting any point of the cylindrical body.

In an alternative embodiment, the cross-section of the trocar is a geometric figure other than a circle and the at least one actuation direction of the laser goes longitudinally through the trocar, from end to end, without intersecting any point of the main body.

In one embodiment, *the laser cutting surgical robot comprises a robotic arm controlled by the central processing unit for the positioning and orientation of the laser cutting module.*

Throughout the description, a robotic arm will be understood to mean an articulated mechanical arm which receives orders from the central processing unit for positioning and/or orienting the laser cutting module in a three-dimensional manner.

In one embodiment, *the surgical robot comprises a viewing optic of the surgical field.*

As a result of the viewing optic, visual information about the surgical region is provided in real time, and this information can be shown to the user or medical personnel through display means, for example, a monitor. This embodiment is particularly important when the actual user or medical personnel decides to directly control the laser through the central processing unit, i.e., the user orders the central processing unit to send particular activation/deactivation and positioning and/or orientation instructions to the laser cutting module of the robot. In these cases, the actual central processing unit can inhibit the laser beam if the manual position and/or orientation are not suitable.

Preferably, the laser cutting module and the viewing optic are previously calibrated. Thus, the central processing unit knows the match existing between the different points of the surgical region where the laser impacts and the different pixels of the images of said surgical region shown by the viewing optic. It is thereby assured that the images shown match with the tissues on which the laser is going to act, even when the central axis of the viewing optic does not coincide with the center of actuation of the laser cutting module.

In one embodiment, *the central processing unit is adapted for performing the positioning and*/*or orientation of the laser cutting module with respect to the empty inner space of the main body of the surgical access port in a continuous manner in order to maintain a real-time tracking of said empty inner space.* In another alternative embodiment, *the central processing unit is adapted for performing the positioning and*/*or orientation of the laser cutting module with respect to the empty inner space of the main body of the surgical access port in an intermittent manner.*

These embodiments contemplate the positioning and/or orientation of the laser being performed in a continuous manner, in order to perform a real-time tracking of the surgical access port, or in an intermittent manner, that is, at a specific instant in time or in a plurality of discrete instants in time. In this second case, a particular positioning and/or orientation frequency can be previously established, or it can be determined that the positioning and/or the orientation are performed when required by the user or medical personnel.

In one embodiment, *the central processing unit controls the three-dimensional position and*/*or orientation of the laser cutting module in order to incise a surgical region through the surgical access port taking the three-dimensional pose of the empty inner space of the main body of the surgical access port and the shape of said empty inner space of the main body of the surgical access port of the numerical model as a reference.*

The cutting performed by the robot must be able to reach different points of the surgical region, i.e., a particular area. This particular area is defined by the actual dimensions, i.e., the shape, of the empty inner space of the main body of the trocar, which are known by the central processing unit.

The actuation of the laser in the surgical region may require the central processing unit to determine a plurality of actuation directions of the laser, such that said central processing unit positions and/or orients the laser cutting module in a three-dimensional manner following a sequence according to said actuation directions.

In one embodiment, the central processing unit defines safety margins with respect to the walls of the main body of the trocar. Said central processing unit thus determines the different actuation directions such that the laser beam does not cross the margins it has defined.

Alternatively, once the laser is positioned and oriented according to a determined actuation direction, the actuation of said laser in the surgical region may require strategies for scanning or for following a defined itinerary across the surgical region, such that the laser can act on the different points of the target tissues.

In one example, the laser cutting module further comprises a scanner and/or an optical system. In this example, once the laser cutting module is oriented and positioned according to an actuation direction determined by the central processing unit, said module, controlled by the central processing unit, positions and/or orients the laser beam in different areas of the surgical region by means of the scanner and/or optical system according to a strategy for scanning or following a previously defined itinerary, provided that said laser beam does not intersect any point of the main body of the trocar.

In another alternative example, the laser cutting surgical system further comprises a galvo system, which is independent of the surgical robot, adapted for positioning the laser beam in different areas of the surgical region once the laser cutting module has been positioned and/or oriented according to an actuation direction by the central processing unit.

In the two preceding examples, as a result of the numerical model comprising the shape of the empty inner space of the main body of the trocar, the central processing unit knows how much the laser can be deviated, both in position and in orientation, with respect to the determined actuation direction in order to be able to incise the different target points of the surgical region without intersecting the main body at any time. Therefore, with the trocar always being referenced, the itinerary of the laser can be scanned or followed in a manner that is always compatible with the three-dimensional pose of the trocar.

In one embodiment, the central processing unit defines safety margins with respect to the walls of the main body of the trocar which the laser beam, oriented and/or positioned by the scanner and/or the optical system or by the galvo system, cannot cross.

In one embodiment, *the numerical model further comprises the shape of the surgical access port.*

As a result of this embodiment, the central processing unit knows the shape of the surgical access port, i.e., it knows the limits in all directions of the main body and of the fiducial markers. As a result of this information, the central processing unit can predict conditions of mechanical interference between the trocar and external elements with respect to same, for example, the robotic arm. In such case, to prevent collisions, the central processing unit stops the movement of said robotic arm.

In one embodiment, *the surgical system comprises a third plurality of fiducial markers; wherein*
- *the third plurality of fiducial markers comprises at least one fixing element configured for being fixed to the patient;*
- *the tracking device is further adapted for establishing the three-dimensional pose in space of the third plurality of fiducial markers for tracking the movements of the patient.*

This embodiment contemplates the tracking device also tracks the movements of the patient, such as movements due to breathing. To that end, it again uses fiducial markers placed on the actual patient in a fixed manner by means of at least one fixing element, for example by means of clips which are firmly attached in an organ or tissue of the patient, such as the spine.

In a particular example in which registration of the patient is performed, the information of the three-dimensional pose of the fiducial markers obtained by the tracking device, together with the visual information provided by the images used for the registration of the patient, allow the central processing unit to determine a desired position of the trocar in the patient.

One way to register the patient consists of taking, before the operation, a computerized axial tomography image or several 2D X-rays of the patient from different viewpoints. The precise shape of the patient's spinal column, or at least of a set of vertebrae on which the fixing element with the fiducial markers, such as the previously mentioned clip, is going to be fixed, is determined based on these images. Subsequently, on the day of the operation, the clip is fixed on the spine of the patient and images are captured again, for example, by means of a plurality of X-rays acquired from different views, such that these images now show the clip and its fiducial markers. In this case, it is important for the fiducial markers to be radiopaque.

The process of registering the patient uses the shape of the vertebrae as a common reference between the first set of images and the second set of images such that when they are matched up, the information from one set can be related with the information from the other set.

In particular, before the operation, the cutting strategy on the first set of images is defined. By matching up the vertebrae of the first set of images and the second set of images, the cutting strategy is related with the position of the fiducial markers located in the clip.

Thus, once the central processing unit determines said desired position of the trocar established by the cutting strategy, the user or medical personnel fix the trocar, for example, to the operating table, and the central processing unit aligns the laser cutting module with said trocar such that the laser acts through the empty inner space of the main body of the trocar. Advantageously, this method ensures optimal laser-trocar alignment.

Alternatively, the central processing unit positions the laser cutting module on the target position of the patient using the information of the patient through the position of the third plurality of markers, for example located in a clip fixed to the spine. Subsequently, the trocar is placed on the patient using the information of the three-dimensional pose of the fiducial markers of the patient obtained by the tracking device, and optionally, using the same visual information of the registration. To that end, the central processing unit provides the medical personnel responsible for placing the trocar with visual information, preferably by means of a 3D model of the trocar in the anatomical context of the patient and/or in the context of the robot, and/or with numerical information, for example, values of distance and angles with respect to the target position of the patient. Advantageously, this method allows for greater precision in the placement of the robot above the patient.

In any of the two preceding alternatives, the quantification of the error committed in the alignment between the laser cutting module and the main body of the trocar is provided; the central processing unit thus repositions and/or reorients the laser cutting module in the case of misalignment.

The use of these fiducial markers in the patient further entails the advantage of detecting if the relative position between the trocar and the actual patient has changed over time. To that end, the central processing unit receives from the tracking device the three-dimensional pose in space of the fiducial markers positioned in the patient and establishes the relative three-dimensional poses between the main body of the trocar and said markers. If these relative three-dimensional poses are modified over time, it means that the markers and/or the actual trocar have experienced an undesired movement. In these cases, the central processing unit preferably gives off an alarm so that the markers and/or the trocar can be repositioned and/or reoriented.

In one example, the fiducial markers of the patient can be fixed by means of clips comprising a flexible articulation so that, in the case of being hit, said articulation prevents the stress caused by being hit from directly affecting the fixing with the patient, and therefore, prevents the clip from coming loose and causing an undesired movement of the markers. In this case, placing the flexible articulation in its initial position again would be sufficient.

In one embodiment, *the central processing unit is further adapted for performing the three-dimensional positioning and*/*or orientation of the laser cutting module with respect to the movement of the patient through the movement of the third plurality of fiducial markers.*

Once the tracking device has established the three-dimensional pose in space of the fiducial markers of the patient, this information is transferred to the central processing unit. As a result of this information, the central processing unit determines that the three-dimensional pose of the trocar may have changed and is therefore misaligned with respect to the laser cutting module of the robot.

In this situation, the central processing unit orders the repositioning and the reorientation of the laser cutting module according to the at least one previously determined actuation direction.

In a particular example, if the alignment error exceeds a limit defined as being acceptable, the central processing unit gives off an alarm. Preferably, an alignment is considered to be unacceptable when the laser beam intersects the main body of the trocar at any point. In another particular example, which is an alternative to or simultaneous with the preceding example, the central processing unit gives off an alarm when it detects that the alignment error cannot be corrected, for example, because there is a failure in communication between said unit and the robot. Preferably, under these circumstances the emission of the laser is stopped.

In one embodiment, *the numerical model further comprises the coordinates of the third plurality of fiducial markers and the relationship of coordinates and orientation between the at least one actuation direction of the laser beam and the third plurality of fiducial markers.*

For the central processing unit to perform the repositioning and/or the reorientation of the laser cutting module, the numerical model must comprise the coordinates of the fiducial markers of the patient and the relationship of coordinates between said fiducial markers and the at least one actuation direction of the laser beam.

### DESCRIPTION OF THE DRAWINGS

These and other features and advantages of the invention will become more apparent based on the following detailed description of a preferred embodiment given solely by way of illustrative and non-limiting example with reference to the attached figures.
Figure 1 shows an embodiment of the surgical access port comprising four fiducial markers.
Figure 2 illustrates an embodiment of the laser cutting surgical system, in which the tracking device is mounted on the robot.
Figure 3 shows an embodiment of the laser cutting surgical system, in which the tracking device visualizes the fiducial markers of the robot and of the surgical access port.
Figure 4 shows an embodiment of the laser cutting surgical system, in which the tracking device visualizes the fiducial markers of the robot, of the surgical access port, and of the patient.

### DETAILED DESCRIPTION OF THE INVENTION

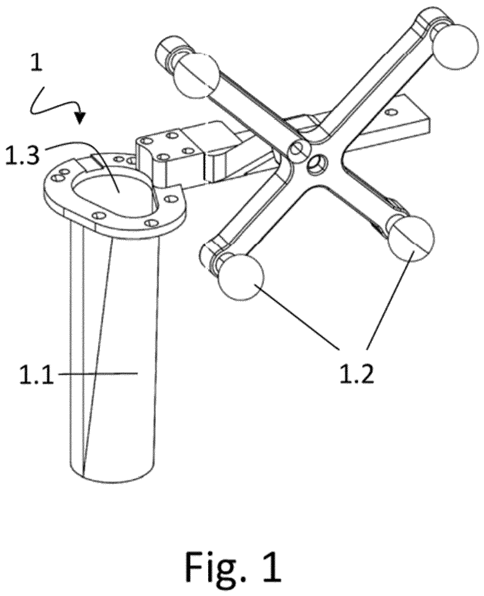

Figure 1 shows an embodiment of the trocar (1) comprising a tubular main body (1.1) with an empty inner space (1.3) therein and four spherical fiducial markers (1.2). These markers (1.2) are spaced from one another, located at the ends of a cross. In this example, the cross is attached to a support which serves for fixing the trocar (1) once it has been inserted into the patient's body. Preferably, the trocar (1) is fixed to the operating table.

In an alternative example, also shown in Figure 1, the fiducial markers (1.2) are not fixed in the form of a cross but rather the holes of the main body (1.1) of the trocar (1), located in the flange arranged at the upper end of said main body (1.1), serve for establishing the fiducial markers (1.2) by means of a tracked probe.

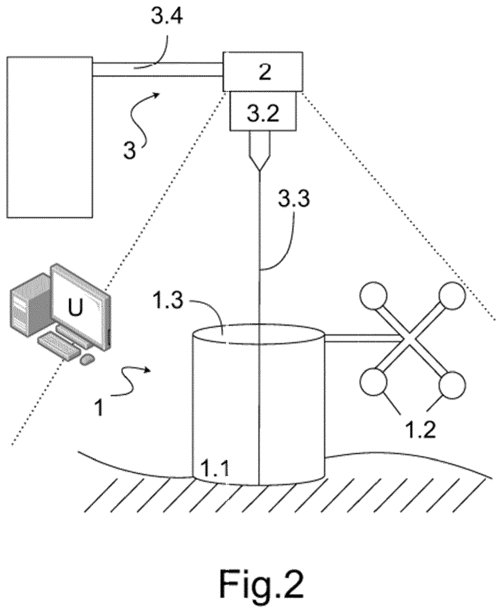

Figure 2 illustrates an embodiment of a laser cutting surgical system comprising a positioning system for a surgical environment and a laser cutting surgical robot (3).

The positioning system, in turn, comprises a surgical access port (1) like the one shown in Figure 1, a tracking device (2) which establishes the three-dimensional pose in space of the fiducial markers (1.2) of the trocar (1), and a central processing unit (U). This central processing unit (U) receives from the tracking device (2) the three-dimensional pose of the fiducial markers (1.2) of the trocar (1) and determines the three-dimensional pose of the empty inner space (1.3) of the main body (1.1) of the surgical access port (1) with respect to a reference coordinate system based on this information. Additionally, the central processing unit stores the reference coordinate system and the three-dimensional pose of the empty inner space (1.3) of the main body (1.1) with respect to the reference coordinate system.

In turn, the laser cutting surgical robot (3) comprises a laser cutting module (3.2) adapted for emitting a laser beam which acts through the empty space (1.3) of the main body (1.1) of the surgical access port (1).

In order to align the beam laser with the trocar (1), the central processing unit (U) additionally determines, based on the three-dimensional pose of the empty inner space (1.3) of the main body (1.1), at least one actuation direction (3.3) of the laser beam such that the laser beam goes through the empty inner space (1.3) of the main body (1.1) in the at least one actuation direction (3.3) without intersecting said main body (1.1). Furthermore, the central processing unit (U) positions and orients the laser cutting module (3.2) for the laser beam to be oriented according to the at least one actuation direction (3.3) it has previously determined. In this particular example, the laser cutting surgical robot (3) comprises a robotic arm (3.4) which is controlled by the central processing unit (U) for the positioning and orientation of the laser cutting module (3.2).

The estimation of the three-dimensional pose of the empty inner space (1.3) of the main body (1.1) of the trocar (1) is performed based on geometric relationships from a numerical model generated by the central processing unit (U). This numerical model comprises at least the coordinates of the fiducial markers (1.2) of the trocar (1) with respect to the reference coordinate system and the relationship of coordinates and orientation between the empty space (1.3) of the main body (1.1) of the surgical access port (1) and said fiducial markers (1.2). The numerical model further comprises the shape of the empty inner space (1.3) of the main body (1.1) of the surgical access port (1). Optionally, the numerical model also comprises the shape of the surgical access port (1).

The geometric relationships which allow the three-dimensional pose of the empty inner space (1.3) of the trocar (1) to be estimated can be determined by means of an *in vivo* calibration by mounting and removing the fiducial markers in/from the trocar as needed, using a tracked probe for that purpose.

Furthermore, it should be mentioned that the positioning and orientation of the laser cutting module (3.2) with respect to the empty inner space (1.3) of the main body (1.1) of the surgical access port (1) can be done either in a continuous manner, in order to maintain a real-time tracking of said empty inner space (1.3), or in an intermittent manner, whether by previously establishing a particular frequency or when required by the user or medical personnel.

Moreover, as seen in this Figure 2, the laser cutting module (2) is mounted on the robot (3), in particular, on the laser cutting module (3.2). This means that the tracking device (2) knows at all times the three-dimensional pose of the laser cutting module (2) and transfers this information to the central processing unit (U) for the unit to determine whether or not the laser and the trocar are aligned and to thus act accordingly.

As for the trocar (1), in this example of Figure 2, it can be seen that the main body (1.1) is cylindrical and the actuation direction (3.3) determined by the central processing unit (U) extends along the axis of revolution of said cylinder.

Optionally, the robot (3) comprises a viewing optic of the surgical field in order to be able to offer to the user or medical personnel an image of said surgical field in real time.

Lastly, the central processing unit (U) can be further adapted for controlling the three-dimensional position and/or orientation of the laser cutting module (3.2) in order to incise a surgical region through the surgical access port (1) taking the three-dimensional pose of the empty inner space (1.3) of the main body (1.1) of the surgical access port (1) and the shape of said empty inner space (1.3) of the main body (1.1) of the surgical access port (1) of the numerical model as a reference.

In one example, the central processing unit (U) controls different three-dimensional positions and/or orientations of the laser cutting module (3.2) for the laser beam to act sequentially according to a plurality of different actuation directions (3.3), the laser beam thus striking a particular surgical region. In another example, the central processing unit (U) controls the laser cutting module (3.2) which is positioned and/or oriented according to a particular actuation direction (3.3) while the laser strikes different points of the surgical region as a result of the laser cutting module (3.2) comprising a scanner and/or an optical system for orienting and/or positioning the laser. Alternatively, the orientation and/or positioning of the laser are performed by means of a galvo system, which is independent of the robot.

In these examples, the central processing unit (U) estimates the available actuation space of the laser, that is, the surgical region which may or may not include safety margins, as a result of knowing the dimensions of the empty inner space (1.3) of the main body (1.1) and the volume in which the rays of the laser beam are confined.

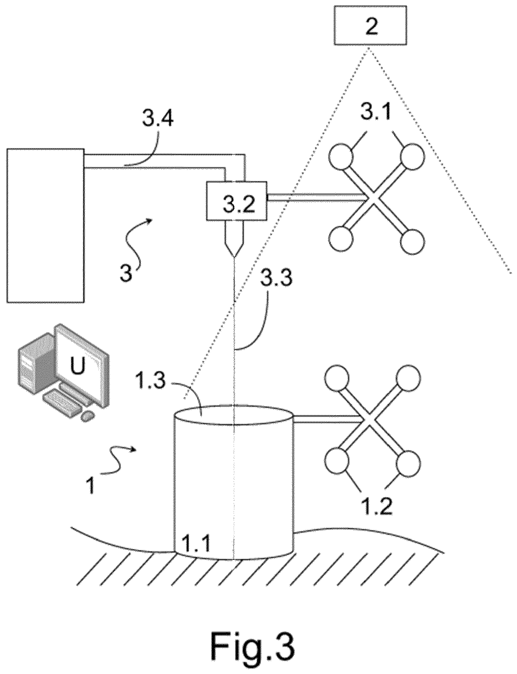

Figure 3 shows another embodiment of the laser cutting surgical system. The difference between this example and the one shown in Figure 2 is that the laser cutting surgical robot (3) further comprises fiducial markers (3.1) and the tracking device (2) is located such that it visualizes both the fiducial markers (3.1) of the robot (3) and the fiducial markers (1.2) of the trocar (1). The remaining features described in the example of Figure 2 can be extrapolated to this embodiment.

In this case, the tracking device (2) does not directly know the three-dimensional pose of the laser cutting module (3.2) but rather estimates the three-dimensional pose of the fiducial markers (3.1) of the robot and sends this information to the central processing unit (U). The central processing unit (U) thus determines the relative three-dimensional pose between the laser cutting module (3.2) and the main body (1.1) of the surgical access port (1), in particular, the empty inner space (1.3), based on this information and stores it.

The estimation of the three-dimensional pose of the laser cutting module (3.2) of the surgical robot (3) is performed based on geometric relationships from the numerical model generated by the central processing unit (U) which, in this example, further comprises the coordinates of the fiducial markers (3.1) of the robot with respect to the reference coordinate system and the relationship of coordinates and orientation between the at least one actuation direction (3.3) of the laser beam and said fiducial markers (3.1).

The geometric relationships which allow the three-dimensional pose of the laser cutting module (3.2) to be estimated can be determined by means of hand-eye calibration, by means of calibration based on geometric restrictions, or by means of direct measurement performed by a metrology system or a calibration device.

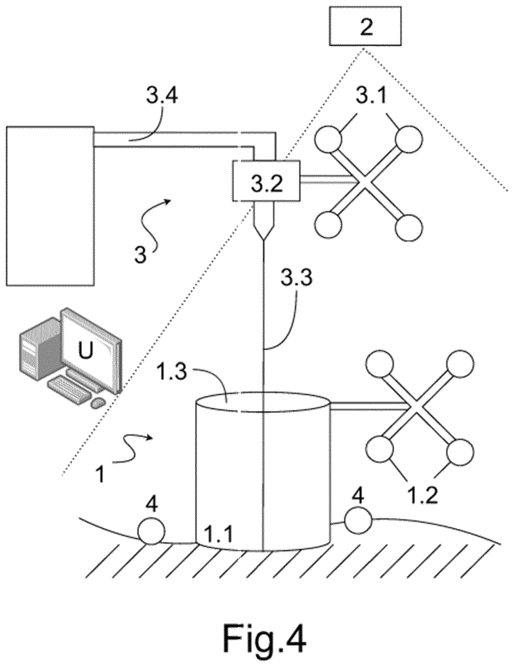

Figure 4 shows another embodiment of the laser cutting surgical system. In this example, the system shown in Figure 3 further comprises fiducial markers (4) arranged in the patient by means of fixing elements. In an example not shown in the figure, the fixing elements are clips fixing the fiducial markers to a tissue of the patient, for example, to the spinous process of the vertebra of the patient. The tracking device (2) is positioned for visualizing all the fiducial markers (1.2, 3.1, 4) at the same time and is further adapted for establishing the three-dimensional pose in space of these fiducial markers (4) of the patient for tracking the movements of said patient.

The central processing unit (U) receives from the tracking device (2) the three-dimensional pose in space of the fiducial markers (4) of the patient and establishes the relative three-dimensional pose between the empty inner space (1.3) of the main body (1.1) of the trocar (1) and said markers (4). If this relative three-dimensional pose is modified over time, it means that the markers (4) and/or the actual trocar (1) have experienced an undesired movement.

Additionally, as a result of this information, the central processing unit (U) knows if the three-dimensional pose of the trocar has changed, and if it is therefore misaligned with respect to the laser cutting module (3.2) of the robot (3). If such misalignment exists, the central processing unit (U) performs the necessary repositioning and/or the reorientation of the laser cutting module (3.2).

In this example, the numerical model further comprises the coordinates of the fiducial markers (4) of the patient and the relationship of coordinates and orientation between the at least one actuation direction (3.3) of the laser beam and said fiducial markers (4).

In an alternative example, the system of the embodiment shown in Figure 2 further comprises fiducial markers (4) fixed to the patient by fixing means, such as one or more clips fixed to one or more vertebrae of the patient, and the tracking device simultaneously visualizes the fiducial markers (4) of the patient and the fiducial markers (1.2) of the trocar (1).

## Claims

1. A laser cutting surgical system, comprising:
- a positioning system for a surgical environment comprising:
• a surgical access port (1) suitable for surgical interventions, comprising:
o a main body (1.1) configured as a tubular conduit, with an empty inner space (1.3) therein and open at the ends of the tubular conduit, and
o a first plurality of fiducial markers (1.2) spaced from one another, and fixed to the main body (1.1) in a rigid manner;
• a tracking device (2) adapted for establishing the three-dimensional pose in space of the first plurality of fiducial markers (1.2) of the surgical access port (1);
• a central processing unit (U) adapted for:
storing a reference coordinate system;
receiving the three-dimensional pose of the first plurality of fiducial markers (1.2) from the tracking device (2);
determining at least the three-dimensional pose of the empty inner space (1.3) of the main body (1.1) of the surgical access port (1) with respect to the reference coordinate system based on the three-dimensional pose of the first plurality of fiducial markers (1.2); and
storing said three-dimensional pose of the empty inner space (1.3) of the main body (1.1) of the surgical access port (1) with respect to the reference coordinate system;
- a laser cutting surgical robot (3) comprising a laser cutting module (3.2) adapted for emitting a laser beam acting throughout the empty inner space (1.3) of the main body (1.1) of the surgical access port (1),
wherein the central processing unit (U) is further adapted for:
determining, based on the three-dimensional pose of the empty inner space (1.3) of the main body (1.1), at least one actuation direction (3.3) of the laser beam such that the laser beam goes through the empty inner space (1.3) of the main body (1.1) in the at least one actuation direction (3.3) without intersecting said main body (1.1);
positioning and orienting the laser cutting module (3.2) for the laser beam to be oriented according to the at least one determined actuation direction (3.3);
wherein the laser cutting surgical system further comprises a third plurality of fiducial markers (4), wherein
- the third plurality of fiducial markers (4) comprises at least one fixing element configured for being fixed to the patient;
- the tracking device (2) is further adapted for establishing the three-dimensional pose in space of the third plurality of fiducial markers (4) for tracking the movements of the patient; and
**characterised in that** the central processing unit (U) is further adapted for performing the three-dimensional positioning and/or orientation of the laser cutting module (3.2) with respect to the movement of the patient through the movement of the third plurality of fiducial markers (4).

2. The laser cutting surgical system according to the preceding claim, wherein:
- the laser cutting surgical robot (3) further comprises a second plurality of fiducial markers (3.1),
- the tracking device (2) is further adapted for establishing the three-dimensional pose in space of the second plurality of fiducial markers (3.1); and
- the central processing unit (U) is further adapted for:
receiving the three-dimensional pose of the second plurality of fiducial markers (3.1) from the tracking device (2);
determining at least the relative three-dimensional pose between the laser cutting module (3.2) and the empty inner space (1.3) of the main body (1.1) of the surgical access port (1) based on the three-dimensional pose of the second plurality of fiducial markers (3.1); and
storing said relative three-dimensional pose between the laser cutting module (3.2) and the empty inner space (1.3) of the main body (1.1) of the surgical access port (1).

3. The surgical system according to any of claims 1 or 2, wherein the central processing unit (U) generates a numerical model comprising at least:
- the shape of the empty inner space (1.3) of the main body (1.1) of the surgical access port (1);
- the coordinates of the first plurality of fiducial markers (1.2) with respect to the reference coordinate system and the relationship of coordinates and orientation between the empty inner space (1.3) of the main body (1.1) of the surgical access port (1) and the first plurality of fiducial markers (1.2); and
- if the system comprises the second plurality of fiducial markers (3.1), the coordinates of the second plurality of fiducial markers (3.1) with respect to the reference coordinate system and the relationship of coordinates and orientation between the at least one actuation direction (3.3) of the laser beam and the second plurality of fiducial markers (3.1).

4. The surgical system according to any of claims 1 to 3, wherein the tubular conduit of the main body (1.1) of the surgical access port (1) is cylindrical.

5. The surgical system according to any of claims 1 to 4, wherein the laser cutting surgical robot (3) comprises a robotic arm (3.4) controlled by the central processing unit (U) for the positioning and orientation of the laser cutting module (3.2).

6. The surgical system according to any of claims 1 to 5, wherein the surgical robot (3) comprises a viewing optic of the surgical field.

7. The surgical system according to any of claims 1 to 6, wherein the central processing unit (U) is adapted for performing the positioning and/or orientation of the laser cutting module (3.2) with respect to the empty inner space (1.3) of the main body (1.1) of the surgical access port (1) in a continuous manner in order to maintain a real-time tracking of said empty inner space (1.3).

8. The surgical system according to any of claims 1 to 6, wherein the central processing unit (U) is adapted for performing the positioning and/or orientation of the laser cutting module (3.2) with respect to the empty inner space (1.3) of the main body (1.1) of the surgical access port (1) in an intermittent manner.

9. The surgical system according to claim 3 and any of claims 1-2, 4-8, wherein the central processing unit (U) controls the three-dimensional position and/or orientation of the laser cutting module (3.2) in order to incise a surgical region through the surgical access port (1) taking the three-dimensional pose of the empty inner space (1.3) of the main body (1.1) of the surgical access port (1) and the shape of said empty inner space (1.3) of the main body (1.1) of the surgical access port (1) of the numerical model as a reference.

10. The surgical system according to claim 3 and any of claims 1-2, 4-9, wherein the numerical model further comprises the shape of the surgical access port (1).

11. The surgical system according to claim 3 and any of claims 1-2, 4-10, wherein the central processing unit (U) determines the three-dimensional pose of the empty inner space (1.3) of the main body (1.1) of the surgical access port (1) by means of the relationships of coordinates and orientation between the empty inner space (1.3) of the main body (1.1) of the surgical access port (1) and the first plurality of fiducial markers (1.2) of the numerical model.

12. The surgical system according to the previous claim, wherein the relationship of coordinates and orientation between the empty inner space (1.3) of the main body (1.1) of the surgical access port (1) and the first plurality of fiducial markers (1.2) is determined by means of a calibration using a tracked probe.

13. The surgical system according to claim 3 and any of claims 1-2, 4-12, wherein the relationships of coordinates and orientation between the at least one actuation direction (3.3) of the laser beam and the second plurality of fiducial markers (3.1) of the numerical model are determined by means of:
- a hand-eye calibration, or
- a calibration based on geometric restrictions, or
- a direct measurement performed by a metrology system or a calibration device.

14. The surgical system according to claim 3 and any of claims 1-2, 4-13, wherein the numerical model further comprises the coordinates of the third plurality of fiducial markers (4) and the relationship of coordinates and orientation between the at least one actuation direction (3.3) of the laser beam and the third plurality of fiducial markers (4).

## Patentansprüche

1. Ein chirurgisches Laserschneidsystem, umfassend:
- ein Positionierungssystem für eine chirurgische Umgebung umfassend:
• einen chirurgischen Zugangsport (1), der für chirurgische Eingriffe geeignet ist, umfassend:
∘ einen Hauptkörper (1.1), der als röhrenförmige Leitung ausgebildet ist, mit einem leeren Innenraum (1.3) darin, der an den Enden der röhrenförmigen Leitung offen ist, und
∘ eine erste Vielzahl von Fiduzialmarkern (1.2), die voneinander beabstandet und starr am Hauptkörper (1.1) befestigt sind;
• eine Tracking-Vorrichtung (2), die dafür ausgelegt ist, die dreidimensionale Lage im Raum der ersten Vielzahl von Fiduzialmarkern (1.2) des chirurgischen Zugangsports (1) festzustellen;
• eine zentrale Verarbeitungseinheit (U), die ausgelegt ist zum:
Speichern eines Referenzkoordinatensystems;
Empfangen der dreidimensionalen Lage der ersten Vielzahl von Fiduzialmarkern (1.2) von der Tracking-Vorrichtung (2);
Bestimmen mindestens der dreidimensionalen Lage des leeren Innenraums (1.3) des Hauptkörpers (1.1) des chirurgischen Zugangsports (1) in Bezug auf das Referenzkoordinatensystem, basierend auf der dreidimensionalen Lage der ersten Vielzahl von Fiduzialmarkern (1.2); und
Speichern der besagten dreidimensionalen Lage des leeren Innenraums (1.3) des Hauptkörpers (1.1) des chirurgischen Zugangsports (1) in Bezug auf das Referenzkoordinatensystem;
- einen chirurgischen Laserschneidroboter (3), der ein Laserschneidmodul (3.2) umfasst, das zum Emittieren eines Laserstrahls ausgelegt ist, der durch den gesamten leeren Innenraum (1.3) des Hauptkörpers (1.1) des chirurgischen Zugangsports (1) wirkt,
wobei die zentrale Verarbeitungseinheit (U) ferner ausgelegt ist zum:
Bestimmen, basierend auf der dreidimensionalen Lage des leeren Innenraums (1.3) des Hauptkörpers (1.1), mindestens einer Betätigungsrichtung (3.3) des Laserstrahls, sodass der Laserstrahl in der mindestens einen Betätigungsrichtung (3.3) durch den leeren Innenraum (1.3) des Hauptkörpers (1.1) verläuft, ohne den besagten Hauptkörper (1.1) durchzuschneiden;
Positionieren und Ausrichten des Laserschneidmoduls (3.2), sodass der Laserstrahl gemäß der mindestens einen bestimmten Betätigungsrichtung (3.3) ausgerichtet ist;
wobei das chirurgische Laserschneidsystem ferner eine dritte Vielzahl von Fiduzialmarkern (4) umfasst, wobei
- die dritte Vielzahl von Fiduzialmarkern (4) mindestens ein Befestigungselement umfasst, das zur Befestigung am Patienten konfiguriert ist;
- die Tracking-Vorrichtung (2) ferner dafür ausgelegt ist, die dreidimensionale Lage im Raum der dritten Vielzahl von Fiduzialmarkern (4) zur Nachverfolgung der Bewegungen des Patienten festzustellen; und
**dadurch gekennzeichnet, dass** die zentrale Verarbeitungseinheit (U) ferner dafür ausgelegt ist, die dreidimensionale Positionierung und/oder Ausrichtung des Laserschneidmoduls (3.2) in Bezug auf die Bewegung des Patienten durch die Bewegung der dritten Vielzahl von Fiduzialmarkern (4) durchzuführen.

2. Das chirurgisches Laserschneidsystem nach dem vorhergehenden Anspruch, wobei:
- der chirurgische Laserschneidroboter (3) ferner eine zweite Vielzahl von Fiduzialmarkern (3.1) umfasst,
- die Tracking-Vorrichtung (2) ferner dafür ausgelegt ist, die dreidimensionale Lage im Raum der zweiten Vielzahl von Fiduzialmarkern (3.1) festzustellen; und
- die zentrale Verarbeitungseinheit (U) ferner ausgelegt ist zum:
Empfangen der dreidimensionalen Lage der zweiten Vielzahl von Fiduzialmarkern (3.1) von der Tracking-Vorrichtung (2);
Bestimmen mindestens der relativen dreidimensionalen Lage zwischen dem Laserschneidmodul (3.2) und dem leeren Innenraum (1.3) des Hauptkörpers (1.1) des chirurgischen Zugangsports (1) basierend auf der dreidimensionalen Lage der zweiten Vielzahl von Fiduzialmarkern (3.1); und
Speichern der besagten relativen dreidimensionalen Lage zwischen dem Laserschneidmodul (3.2) und dem leeren Innenraum (1.3) des Hauptkörpers (1.1) des chirurgischen Zugangsports (1).

3. Das chirurgisches System nach einem der Ansprüche 1 oder 2, wobei die zentrale Verarbeitungseinheit (U) ein numerisches Modell erzeugt, das mindestens umfasst:
- die Form des leeren Innenraums (1.3) des Hauptkörpers (1.1) des chirurgischen Zugangsports (1);
- die Koordinaten der ersten Vielzahl von Fiduzialmarkern (1.2) in Bezug auf das Referenzkoordinatensystem und die Beziehung der Koordinaten und Ausrichtung zwischen dem leeren Innenraum (1.3) des Hauptkörpers (1.1) des chirurgischen Zugangsports (1) und der ersten Vielzahl von Fiduzialmarkern (1.2); und
- wenn das System die zweite Vielzahl von Fiduzialmarkern (3.1) umfasst, die Koordinaten der zweiten Vielzahl von Fiduzialmarkern (3.1) in Bezug auf das Referenzkoordinatensystem und die Beziehung der Koordinaten und Ausrichtung zwischen der mindestens einen Betätigungsrichtung (3.3) des Laserstrahls und der zweiten Vielzahl von Fiduzialmarkern (3.1).

4. Das chirurgisches System nach einem der Ansprüche 1 bis 3, wobei die röhrenförmige Leitung des Hauptkörpers (1.1) des chirurgischen Zugangsports (1) zylindrisch ist.

5. Das chirurgisches System nach einem der Ansprüche 1 bis 4, wobei der chirurgische Laserschneidroboter (3) einen Roboterarm (3.4) umfasst, der von der zentralen Verarbeitungseinheit (U) zur Positionierung und Ausrichtung des Laserschneidmoduls (3.2) gesteuert wird.

6. Das chirurgisches System nach einem der Ansprüche 1 bis 5, wobei der chirurgische Roboter (3) eine Betrachtungsoptik des Operationsfeldes umfasst.

7. Das chirurgisches System nach einem der Ansprüche 1 bis 6, wobei die zentrale Verarbeitungseinheit (U) dafür ausgelegt ist, die Positionierung und/oder Ausrichtung des Laserschneidmoduls (3.2) in Bezug auf den leeren Innenraum (1.3) des Hauptkörpers (1.1) des chirurgischen Zugangsports (1) in einer kontinuierlichen Weise durchzuführen, um eine Echtzeit-Nachverfolgung des besagten leeren Innenraums (1.3) aufrechtzuerhalten.

8. Das chirurgisches System nach einem der Ansprüche 1 bis 6, wobei die zentrale Verarbeitungseinheit (U) dafür ausgelegt ist, die Positionierung und/oder Ausrichtung des Laserschneidmoduls (3.2) in Bezug auf den leeren Innenraum (1.3) des Hauptkörpers (1.1) des chirurgischen Zugangsports (1) in einer intermittierenden Weise durchzuführen.

9. Das chirurgisches System nach Anspruch 3 und einem der Ansprüche 1-2, 4-8, wobei die zentrale Verarbeitungseinheit (U) die dreidimensionale Position und/oder Ausrichtung des Laserschneidmoduls (3.2) steuert, um einen chirurgischen Bereich durch den chirurgischen Zugangsport (1) einzuschneiden, wobei die dreidimensionale Lage des leeren Innenraums (1.3) des Hauptkörpers (1.1) des chirurgischen Zugangsports (1) und die Form des besagten leeren Innenraums (1.3) des Hauptkörpers (1.1) des chirurgischen Zugangsports (1) des numerischen Modells als Referenz genommen werden.

10. Das chirurgisches System nach Anspruch 3 und einem der Ansprüche 1-2, 4-9, wobei das numerische Modell ferner die Form des chirurgischen Zugangsports (1) umfasst.

11. Das chirurgisches System nach Anspruch 3 und einem der Ansprüche 1-2, 4-10, wobei die zentrale Verarbeitungseinheit (U) die dreidimensionale Lage des leeren Innenraums (1.3) des Hauptkörpers (1.1) des chirurgischen Zugangsports (1) mittels der Beziehungen von Koordinaten und Ausrichtung zwischen dem leeren Innenraum (1.3) des Hauptkörpers (1.1) des chirurgischen Zugangsports (1) und der ersten Vielzahl von Fiduzialmarkern (1.2) des numerischen Modells bestimmt.

12. Das chirurgisches System nach dem vorhergehenden Anspruch, wobei die Beziehung der Koordinaten und Ausrichtung zwischen dem leeren Innenraum (1.3) des Hauptkörpers (1.1) des chirurgischen Zugangsports (1) und der ersten Vielzahl von Fiduzialmarkern (1.2) mittels einer Kalibrierung unter Verwendung einer getrackten Sonde bestimmt wird.

13. Das chirurgisches System nach Anspruch 3 und einem der Ansprüche 1-2, 4-12, wobei die Beziehungen der Koordinaten und Ausrichtung zwischen der mindestens einen Betätigungsrichtung (3.3) des Laserstrahls und der zweiten Vielzahl von Fiduzialmarkern (3.1) des numerischen Modells bestimmt werden mittels:
- einer Hand-Auge-Kalibrierung, oder
- einer Kalibrierung basierend auf geometrischen Beschränkungen, oder
- einer direkten Messung, die durch ein Metrologiesystem oder eine Kalibrierungsvorrichtung durchgeführt wird.

14. Das chirurgisches System nach Anspruch 3 und einem der Ansprüche 1-2, 4-13, wobei das numerische Modell ferner die Koordinaten der dritten Vielzahl von Fiduzialmarkern (4) und die Beziehung der Koordinaten und Ausrichtung zwischen der mindestens einen Betätigungsrichtung (3.3) des Laserstrahls und der dritten Vielzahl von Fiduzialmarkern (4) umfasst.

## Revendications

1. Un système chirurgical de coupe au laser, comprenant :
- un système de positionnement pour un environnement chirurgical comprenant :
• un orifice d'accès chirurgical (1) adapté pour des interventions chirurgicales, comprenant :
∘ un corps principal (1.1) configuré comme un conduit tubulaire, ayant un espace intérieur vide (1.3) dans celui-ci et ouvert aux extrémités du conduit tubulaire, et
∘ une première pluralité de repères fiduciaires (1.2) espacés les uns des autres, et fixés au corps principal (1.1) d'une manière rigide,
• un dispositif de suivi (2) adapté pour établir la pose tridimensionnelle dans l'espace de la première pluralité de repères fiduciaires (1.2) de l'orifice d'accès chirurgical (1),
• une unité de traitement centrale (U) adaptée pour :
stocker un système de coordonnées de référence,
recevoir la pose tridimensionnelle de la première pluralité de repères fiduciaires (1.2) à partir du dispositif de suivi (2),
déterminer au moins la pose tridimensionnelle de l'espace intérieur vide (1.3) du corps principal (1.1) de l'orifice d'accès chirurgical (1) par rapport au système de coordonnées de référence sur la base de la pose tridimensionnelle de la première pluralité de repères fiduciaires (1.2), et
stocker ladite pose tridimensionnelle de l'espace intérieur vide (1.3) du corps principal (1.1) de l'orifice d'accès chirurgical (1) par rapport au système de coordonnées de référence,
- un robot chirurgical de coupe au laser (3) comprenant un module de coupe au laser (3.2) adapté pour émettre un faisceau laser agissant dans tout l'espace intérieur vide (1.3) du corps principal (1.1) de l'orifice d'accès chirurgical (1),
dans lequel l'unité de traitement centrale (U) est en outre adaptée pour :
déterminer, sur la base de la pose tridimensionnelle de l'espace intérieur vide (1.3) du corps principal (1.1), au moins une direction d'actionnement (3.3) du faisceau laser de telle sorte que le faisceau laser traverse l'espace intérieur vide (1.3) du corps principal (1.1) dans la au moins une direction d'actionnement (3.3) sans intersecter ledit corps principal (1.1),
positionner et orienter le module de coupe au laser (3.2) pour le faisceau laser à orienter en fonction de la au moins une direction d'actionnement (3.3) déterminée,
dans lequel le système chirurgical de coupe au laser comprend en outre une troisième pluralité de repères fiduciaires (4), dans lequel
- la troisième pluralité de repères fiduciaires (4) comprend au moins un élément de fixation configuré pour être fixé au patient,
- le dispositif de suivi (2) est en outre adapté pour établir la pose tridimensionnelle dans l'espace de la troisième pluralité de repères fiduciaires (4) pour suivre les mouvements du patient, et
**caractérisé en ce que** l'unité de traitement centrale (U) est en outre adaptée pour effectuer le positionnement tridimensionnel et/ou l'orientation du module de coupe au laser (3.2) par rapport au mouvement du patient via le mouvement de la troisième pluralité de repères fiduciaires (4).

2. Le système chirurgical de coupe au laser selon la revendication précédente, dans lequel :
• le robot chirurgical de coupe au laser (3) comprend en outre une deuxième pluralité de repères fiduciaires (3.1),
• le dispositif de suivi (2) est en outre adapté pour établir la pose tridimensionnelle dans l'espace de la deuxième pluralité de repères fiduciaires (3.1), et
• l'unité de traitement centrale (U) est en outre adaptée pour :
recevoir la pose tridimensionnelle de la deuxième pluralité de repères fiduciaires (3.1) à partir du dispositif de suivi (2),
déterminer au moins la pose tridimensionnelle relative entre le module de coupe au laser (3.2) et l'espace intérieur vide (1.3) du corps principal (1.1) de l'orifice d'accès chirurgical (1) sur la base de la pose tridimensionnelle de la deuxième pluralité de repères fiduciaires (3.1), et
stocker ladite pose tridimensionnelle relative entre le module de coupe au laser (3.2) et l'espace intérieur vide (1.3) du corps principal (1.1) de l'orifice d'accès chirurgical (1).

3. Le système chirurgical selon l'une quelconque des revendications 1 ou 2, dans lequel l'unité de traitement centrale (U) génère un modèle numérique comprenant au moins :
- la forme de l'espace intérieur vide (1.3) du corps principal (1.1) de l'orifice d'accès chirurgical (1),
- les coordonnées de la première pluralité de repères fiduciaires (1.2) par rapport au système de coordonnées de référence et la relation de coordonnées et d'orientation entre l'espace intérieur vide (1.3) du corps principal (1.1) de l'orifice d'accès chirurgical (1) et la première pluralité de repères fiduciaires (1.2), et
- si le système comprend la deuxième pluralité de repères fiduciaires (3.1), les coordonnées de la deuxième pluralité de repères fiduciaires (3.1) par rapport au système de coordonnées de référence, et la relation de coordonnées et d'orientation entre la au moins une direction d'actionnement (3.3) du faisceau laser et la deuxième pluralité de repères fiduciaires (3.1).

4. Le système chirurgical selon l'une quelconque des revendications 1 à 3, dans lequel le conduit tubulaire du corps principal (1.1) de l'orifice d'accès chirurgical (1) est cylindrique.

5. Le système chirurgical selon l'une quelconque des revendications 1 à 4, dans lequel le robot chirurgical de coupe au laser (3) comprend un bras robotique (3.4) commandé par l'unité de traitement centrale (U) pour le positionnement et l'orientation du module de coupe au laser (3.2).

6. Le système chirurgical selon l'une quelconque des revendications 1 à 5, dans lequel le robot chirurgical (3) comprend une optique de visualisation du champ opératoire.

7. Le système chirurgical selon l'une quelconque des revendications 1 à 6, dans lequel l'unité de traitement centrale (U) est adaptée pour effectuer le positionnement et/ou l'orientation du module de coupe au laser (3.2) par rapport à l'espace intérieur vide (1.3) du corps principal (1.1) de l'orifice d'accès chirurgical (1) d'une manière continue afin de maintenir un suivi en temps réel dudit espace intérieur vide (1.3).

8. Le système chirurgical selon l'une quelconque des revendications 1 à 6, dans lequel l'unité de traitement centrale (U) est adaptée pour effectuer le positionnement et/ou l'orientation du module de coupe au laser (3.2) par rapport à l'espace intérieur vide (1.3) du corps principal (1.1) de l'orifice d'accès chirurgical (1) d'une manière intermittente.

9. Le système chirurgical selon la revendication 3 et l'une quelconque des revendications 1 à 2 et 4 à 8, dans lequel l'unité de traitement centrale (U) commande la position tridimensionnelle et/ou l'orientation du module de coupe au laser (3.2) afin d'inciser une région chirurgicale à travers l'orifice d'accès chirurgical (1) en prenant comme référence la pose tridimensionnelle de l'espace intérieur vide (1.3) du corps principal (1.1) de l'orifice d'accès chirurgical (1) et la forme dudit espace intérieur vide (1.3) du corps principal (1.1) de l'orifice d'accès chirurgical (1) du modèle numérique.

10. Le système chirurgical selon la revendication 3 et l'une quelconque des revendications 1 à 2 et 4 à 9, dans lequel le modèle numérique comprend en outre la forme de l'orifice d'accès chirurgical (1).

11. Le système chirurgical selon la revendication 3 et l'une quelconque des revendications 1 à 2 et 4 à 10, dans lequel l'unité de traitement centrale (U) détermine la pose tridimensionnelle de l'espace intérieur vide (1.3) du corps principal (1.1) de l'orifice d'accès chirurgical (1) au moyen des relations de coordonnées et d'orientation entre l'espace intérieur vide (1.3) du corps principal (1.1) de l'orifice d'accès chirurgical (1) et la première pluralité de repères fiduciaires (1.2) du modèle numérique.

12. Le système chirurgical selon la revendication précédente, dans lequel la relation de coordonnées et d'orientation entre l'espace intérieur vide (1.3) du corps principal (1.1) de l'orifice d'accès chirurgical (1) et la première pluralité de repères fiduciaires (1.2) est déterminée au moyen d'un calibrage utilisant une sonde suivie.

13. Le système chirurgical selon la revendication 3 et l'une quelconque des revendications 1 à 2 et 4 à 12, dans lequel les relations de coordonnées et d'orientation entre la au moins une direction d'actionnement (3.3) du faisceau laser et la deuxième pluralité de repères fiduciaires (3.1) du modèle numérique sont déterminées au moyen :
• d'un calibrage œil-main, ou
• d'un calibrage basé sur des restrictions géométriques, ou
• d'une mesure directe réalisée par un système de métrologie ou un dispositif de calibrage.

14. Le système chirurgical selon la revendication 3 et l'une quelconque des revendications 1 à 2 et 4 à 13, dans lequel le modèle numérique comprend en outre les coordonnées de la troisième pluralité de repères fiduciaires (4) et la relation de coordonnées et d'orientation entre la au moins une direction d'actionnement (3.3) du faisceau laser et la troisième pluralité de repères fiduciaires (4).
